# EUROPEAN PATENT APPLICATION

(11) **EP 4 464 355 A1**
(43) Date of publication of application: **20.11.2024**
(21) Application number: 23812835.9
(22) Date of filing: 31.03.2023
(51) Int. Cl.: A61M 15/00, A61M 11/00, A61M 31/00

(54) **NEBULIZER, AND METHOD FOR DELIVERING AND ATOMIZING FLUID**

(71) Applicant: CF Pharmtech, Inc., Jiangsu 215143 (CN)
(72) Inventor: TONG, Xuwen, Suzhou, Jiangsu 215143 (CN); BOVET, Lili, Suzhou, Jiangsu 215143 (CN); BLOWER, Henry Benjamin, Suzhou, Jiangsu 215143 (CN); COWEN, Daniel George, Suzhou, Jiangsu 215143 (CN); EADE, Holly Sarah, Suzhou, Jiangsu 215143 (CN); JIMMEZ, Gaspar Rodriguez, Suzhou, Jiangsu 215143 (CN)
(74) Representative: Canzler & Bergmeier Patentanwälte Partnerschaft mbB
(86) International application number: PCT/CN2023/085620
(87) International publication number: WO 2024/197862

(57) **Abstract**

Disclosed are an atomizer for a fluid and a method for conveying and atomizing a fluid. The atomizer includes: a cartridge for accommodating multiple doses of the fluid; a delivery mechanism movable from an initial position to a tensioning position for withdrawal of a medication dosage of the fluid from the cartridge when the atomizer is tensioned, and movable from the tensioning position to the initial position for atomization of the medication dosage of the fluid when the atomizer is activated; a pre-determined amount of the fluid is atomized by a temporary rebound motion of the delivery mechanism during its movement from the initial position to the tensioning position, and the medication dosage is a single medication dose, and the pre-determined amount is less than the medication dosage.

## Description

### TECHNICAL FIELD

The present application relates to the technical field of atomizers, in particular, to an atomizer for a fluid, and a method for conveying and atomizing a fluid.

### BACKGROUND

As medical devices, inhalation atomizers, also known as soft mist inhalers, atomizers, or the like, can be used for the treatment of upper respiratory tract diseases by dispersing a drug liquid into fine particles/aerosol, which are inhaled by users via breathing to allow the drug to enter and deposit in the respiratory tract and lung so as to achieve the purpose of treatment.

When the atomizer is used for the first time or after it has been left for a certain period of time, on the one hand, air or deposit within the internal delivery mechanism thereof will affect the metering accuracy of the atomizer, which will make the dose of the drug inhaled by the user insufficient to produce a good therapeutic effect; on the other hand, the fluid passageway thereof may harbor bacteria or be contaminated, and the drug liquid flowing through the fluid passageway will bring bacteria or contaminants to the user, affecting the physical health of the user.

Therefore, there is an urgent need to provide an atomizer for a fluid, which can deliver drug to a user more accurately and avoid introducing new safety concerns to the user.

### SUMMARY OF THE INVENTION

In view of the above shortcomings of the related technology, the object of the present application is to provide an atomizer for a fluid and a method for conveying and atomizing a fluid to overcome the above technical problems existing in the above related technology.

To achieve the above object and other related objects, in a first aspect, the present application provides an atomizer for a fluid including: a cartridge for accommodating multiple doses of the fluid; and a delivery mechanism movable from an initial position to a tensioning position for withdrawal of a medication dosage of the fluid from the cartridge when the atomizer is tensioned, and movable from the tensioning position to the initial position for atomization of the medication dosage of the fluid when the atomizer is activated; a pre-determined amount of the fluid is atomized by a temporary rebound motion of the delivery mechanism during its movement from the initial position to the tensioning position, and the medication dosage is a single medication dose, and the pre-determined amount is less than the medication dosage.

In some embodiments disclosed in the first aspect of the present application, the atomizer further includes: an atomizer housing for receiving a cartridge; and a lower housing for opening the atomizer to replace or insert the cartridge. A rotational motion of the lower housing relative to the atomizer housing enables the atomizer to be tensioned or ready for use.

In some embodiments disclosed in the first aspect of the present application, the delivery mechanism further includes: a cartridge translator for connecting with the cartridge and driving the cartridge to move in an axial direction; a drive spring coupled with the cartridge translator for driving the cartridge translator back to the initial position when an elastic force is released; and a transmission structure for cooperating with the cartridge translator to convert a rotational motion of the atomizer when being tensioned into an axial movement. During one rotational motion, the axial movement includes a first moving stroke and a second moving stroke, and the temporary rebound motion occurs during switching from the first moving stroke to the second moving stroke.

In some embodiments disclosed in the first aspect of the present application, the one rotational motion is a 180° rotational motion centered on an axis of the atomizer.

In some embodiments disclosed in the first aspect of the present application, the transmission structure includes: a first track including an inclined surface formed on the atomizer housing of the atomizer, the inclined surface being in contact with the cartridge translator to achieve the first moving stroke of the axial movement; and a second track formed on the cartridge translator, the second track being in contact with a blocking member after the cartridge translator is detached from the first track to achieve the second moving stroke of the axial movement.

In some embodiments disclosed in the first aspect of the present application, the blocking member is rotatably arranged on the atomizer housing and pushed to rotate to contact the second track during the rotational motion.

In some embodiments disclosed in the first aspect of the present application, the atomizer housing of the atomizer includes an upper housing part and an inner housing part. The inner housing part is rotatably supported on the upper housing part, and a protruding portion is provided on the inner housing part to push the blocking member to rotate during the rotational motion.

In some embodiments disclosed in the first aspect of the present application, the blocking member is further configured to be rotated by manual operation to release the cartridge translator to allow the drive spring to release an elastic force.

In some embodiments disclosed in the first aspect of the present application, the transmission structure includes at least two first tracks and at least two second tracks.

In some embodiments disclosed in the first aspect of the present application, a groove is provided at an initial position of the second track, When the cartridge translator is detached from the first track, the groove allows the drive spring to release some elastic force to drive the cartridge translator to move upwards until the cartridge translator is blocked by the blocking member, thereby forming the rebound motion.

In some embodiments disclosed in the first aspect of the present application, a depth of the groove is positively correlated with the pre-determined amount or the medication dosage.

In some embodiments disclosed in the first aspect of the present application, the depth of the groove ranges from 0.1 to 1.5 mm.

In some embodiments disclosed in the first aspect of the present application, the cartridge translator includes a guide structure which moves on the first track to achieve the first moving stroke of the axial movement.

In some embodiments disclosed in the first aspect of the present application, the pre-determined amount is configured to rinse a nozzle of the atom izer.

In some embodiments disclosed in the first aspect of the present application, the pre-determined amount ranges from 0.15 to 3 µL.

In some embodiments disclosed in the first aspect of the present application, the pre-determined amount is between 1% and 15% of the medication dosage.

In some embodiments disclosed in the first aspect of the present application, the fluid includes an active substance.

In certain embodiments disclosed in the first aspect of the present application, the active substance includes at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors.

In a second aspect, the present application provides a method for conveying and atomizing a fluid, including: moving a delivery mechanism from an initial position to a tensioning position for withdrawal of a medication dosage of the fluid from a cartridge; generating a temporary rebound motion by the delivery mechanism during its movement from the initial position to the tensioning position for atomization of a pre-determined amount of the fluid; and moving the delivery mechanism from the tensioning position to the initial position for atomization of the medication dosage of the fluid; the medication dosage is a single medication dose, and the pre-determined amount is less than the medication dosage.

In some embodiments disclosed in the second aspect of the present application, the pre-determined amount is configured to rinse a nozzle of the atom izer.

In some embodiments disclosed in the second aspect of the present application, the pre-determined amount ranges from 0.15 to 3 µL.

In some embodiments disclosed in the second aspect of the present application, the pre-determined amount is between 1% and 15% of the medication dosage.

In some embodiments disclosed in the second aspect of the present application, the fluid contains an active substance.

In some embodiments disclosed in the second aspect of the present application, the active substance includes at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors.

In summary, the atomizer for a fluid and the method for conveying and atomizing a fluid as disclosed in the present application conveys and atomizes a pre-determined amount of the fluid during the withdrawal of a medication dosage of the fluid by the atomizer, thereby avoiding any potential health hazard or wrong guidance brought by the atomizer to a patient/user, while ensuring the accuracy of the medication dosage.

Those skilled in the art can easily perceive other aspects and advantages of the present application from the following detailed description. In the following detailed description, only exemplary embodiments of the present application are shown and described. As those skilled in the art will appreciate, the disclosure of the present application enables those skilled in the art to make changes to the specific embodiments as disclosed without departing from the spirit and scope of the invention to which the present application is involved. Correspondingly, the description in the accompanying drawings and the specification of the present application are only exemplary, rather than restrictive.

### BRIEF DESCRIPTION OF DRAWINGS

The specific features of the invention involved in the present application are set forth in the appended claims. By reference to the exemplary embodiments and the accompanying drawings as detailed below, the features and advantages of the invention involved in the present application can be better understood. The accompany drawings are briefly described as follows:
FIG. 1 shows a schematic diagram of generation stage of a pre-determined amount of a fluid in an embodiment of the related art.
FIG. 2 shows a partial schematic structural diagram of an atomizer in an embodiment of the present application.
FIG. 3 shows an exploded schematic structural diagram of the atomizer in the embodiment of the present application as shown in FIG. 2.
FIG. 4 shows a schematic cross-sectional structural diagram of the atomizer in the embodiment of the present application as shown in FIG. 3.
FIG. 5 shows a schematic diagram of generation stage of a pre-determined amount of the fluid in an embodiment of the present application.
FIG. 6 shows an exploded schematic structural diagram of an upper housing part in an embodiment of the present application.
FIG. 7 shows a schematic process diagram of a blocking member being pushed in an embodiment of the present application.
FIG. 8 shows a schematic process diagram of releasing the block of a blocking member on a delivery mechanism in an embodiment of the present application.
FIG. 9 shows a schematic structural diagram of a suction nozzle in combination with a cartridge translator in an embodiment of the present application.
FIG. 10 shows a three-dimensional schematic structural diagram of a suction nozzle in an embodiment of the present application.
FIG. 11 shows a three-dimensional schematic structure diagram of a cartridge translator in an embodiment of the present application.
FIG. 12 shows a flowchart of a method for conveying and atomizing a fluid in an embodiment of the present application.

### DETAILED DESCRIPTION OF THE INVENTION

Hereinafter the embodiments of the present application are described by specific examples, and those skilled in the art can easily understand other advantages and effects of the present application from the disclosure in the description.

In the following description, several embodiments of the present application are described by reference to the accompanying drawings. It is to be understood that other embodiments can be used, and module or unit composition, electrical, and operational changes can be made without departing from the spirit and scope of the present application. The following detailed description should not be considered restrictive, while the scope of the embodiments of the present application is defined only by the claims of the patent publication. The terminology used herein is merely to describe particular embodiments and is not intended to limit the present application.

Although in some instances the terms first, second and the like are used herein to describe various elements or parameters, those elements or parameters should not be limited by these terms. The terms are only used to distinguish one element or parameter from another element or parameter. For example, a first liquid volume state may be called a second liquid volume state, and similarly a second liquid volume state may be called a first liquid volume state, without departing from the scope of the various described embodiments. Both the first liquid volume state and the second liquid volume state are to describe a liquid volume state, but do not involve the same liquid volume state unless otherwise clearly indicated in the context.

Furthermore, as used herein, the singular forms of "a", "an" and "the" are intended to further comprise the plural forms unless otherwise indicated in the context. It should be further understood that the terms "comprise/comprising" and "include/including" indicate the presence of features, steps, operations, elements, components, items, types, and/or groups as described, but do not exclude the presence, occurrence or addition of one or more of other features, steps, operations, elements, components, items, types, and/or groups. The terms "or" and "and/or" as used herein are to be construed as inclusive or mean any one or combination. Thus, "A, B or C" or "A, B and/or C" means "anyone of A; B; C; A and B; A and C; B and C; and A, B and C". An exception to such definition occurs only if a combination of elements, functions, steps, or operations is inherently mutually exclusive in some manners.

To solve the technical problem described in the background that the metering is inaccurate and a potential health hazard is thus caused to the patient/user due to failure of proper dosing when the atomizer is used for the first time or after it has been left for a certain period of time, the atomizer is designed in some embodiments to atomize a pre-determined amount of the fluid to rinse the atomizer prior to each delivery of the medication dosage of fluid. However, in such design, the conveying and atomization of the pre-determined amount of the fluid occurs after the withdrawal (also referred to as suction) of the medication dosage of the fluid. That is to say, the atomizer has to further withdraw the pre-determined amount of the fluid for atomization after the withdrawal of the medication dosage of the fluid. The medication dosage refers to a single medication dosage, which is an amount capable of producing a therapeutic effect on the patient/user. The pre-determined amount refers to a pre-designed amount according to its use, which can be pre-designed by those skilled in the art according to its practical use. Typically, the pre-determined amount is less than the medication dosage.

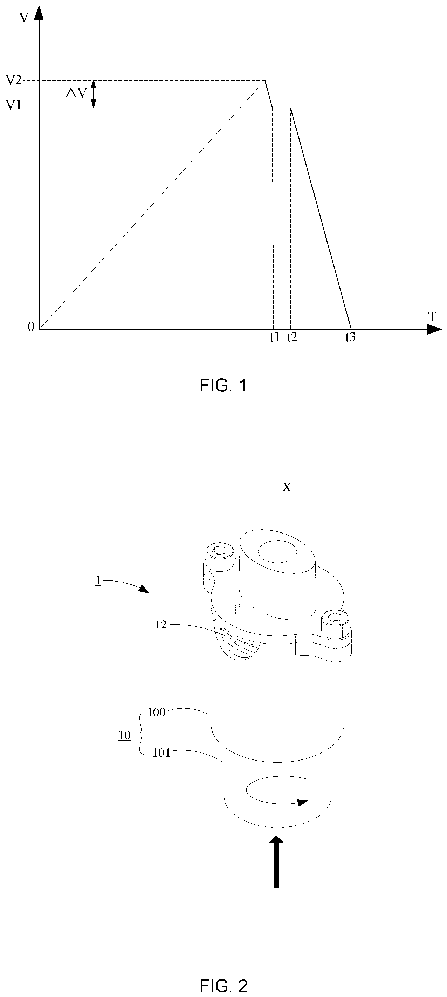

Hereinafter, the manner of atomizing the pre-determined amount of the fluid is described by reference to FIG. 1. FIG. 1 shows a schematic diagram of the generation phase of the pre-determined amount of the fluid in an embodiment of the related art, wherein the abscissa indicates a timeline of triggering and activating the atomizer. Taking the atomizer for conveying and atomizing the fluid by a mechanical force generated by the spring as an example, the ordinate indicates a compression degree, also referred to as the stored energy of the spring, which can reflect the withdrawn and released amounts of the fluid.

As shown in FIG. 1, Stage 0 to t1 involves a triggering stage of the atomizer, which includes two processes: the first process is that the atomizer withdraws a fluid in an amount of exceeding the medication dosage by tensioning the spring (corresponding to V2 in FIG. 1, and V1 corresponding to the medication dosage of the fluid), and the second process is that the spring automatically releases a small part of the compression/energy to convey and atomize the pre-determined amount of the fluid (corresponding to ΔV in FIG. 1). After triggering the atomizer, the atomizer enters a ready-to-use state, which refers to a state in which the atomizer has stored a medication dosage of the fluid by, e.g., a pressure chamber, and is waiting for activation. As shown in FIG. 1, the atomizer in Stage t1 to t2 is in the ready-to-use state. Stage t2 to t3 involves a stage of activating the atomizer. For example, at t2, the patient/user manually operates to activate the atomizer so that the atomizer conveys and atomizes the medication dosage of fluid in Stage t2 to t3.

It should be understood that triggering the atomizer refers to a process of driving the atomizer to withdraw the fluid from a fluid-containing cartridge and enter a ready-to-use stage. According to the main states of different parts in the triggering stage of the atomizer, triggering the atomizer can also be called tensioning the atomizer, stretching the atomizer, rotating the atomizer, or the like. Activating the atomizer refers to a process in which the atomizer is activated to convey and atomize the medication dosage of the fluid. According to the main states of different parts in the stage of activating the atomizer, activating the atomizer can also be called releasing the atomizer, resetting the atomizer, or the like.

In the embodiment as shown in FIG. 1, the process of conveying and atomizing the pre-determined amount of the fluid occurs at the end of the triggering stage of the atomizer, that is, after the withdrawal of the medication dosage of the fluid. It will result in: (1) the release of the pre-determined amount of the fluid automatically occurs at the end of the triggering stage of the atomizer, and further, it automatically occurs after the atomizer withdraws the fluid, which may easily mislead the patient/user into thinking that the atomizer has already started to release the medication dosage. For example, it is required for the patient/user to twist the atomizer for withdrawal of the fluid, and at the end of which the release of the pre-determined amount of the fluid will automatically occurs, and thus the patient/user may easily think that the atomizer has been used; (2) the medication dosage is indirectly obtained by subtracting the pre-determined amount (i.e., V1) from the total withdrawal amount (i.e., V2). In such manner, it has to ensure the accuracy of two amounts so as to ensure the accuracy of the medication dosage, which brings difficulties to design. In addition, due to the repeated use of the atomizer, even if the two amounts are ensured to be accurate in the initial design, various parts of the atomizer will be worn in each process of conveying and atomizing the pre-determined amount of the fluid. Still further, the process of conveying and atomizing the pre-determined amount of the fluid occurs at the end of the triggering stage of the atomizer, wherein the atomizer is generally over-tensioned (i.e., indicating that the atomizer is tensioned to a position at which the fluid can be withdrawn in an amount in excess of the medication dosage). This process will further increase the wear of parts, so that the release of the pre-determined amount will not be so accurate, and thus the medication dosage will not be accurate, thereby failing to achieve a good therapeutic effect.

In view of the foregoing, in some embodiments provided in the present application, an atomizer for a fluid is disclosed, which conveys and atomizes a pre-determined amount of the fluid during the withdrawal of a medication dosage of the fluid by the atomizer, thereby avoiding the health hazard or wrong guidance brought by the atomizer to a patient/user. The atomizer provided in the present application can also ensure the accuracy of the medication dosage in a user/patient.

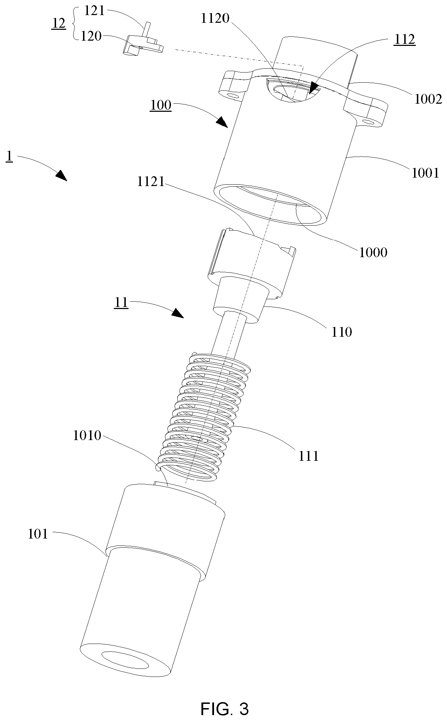

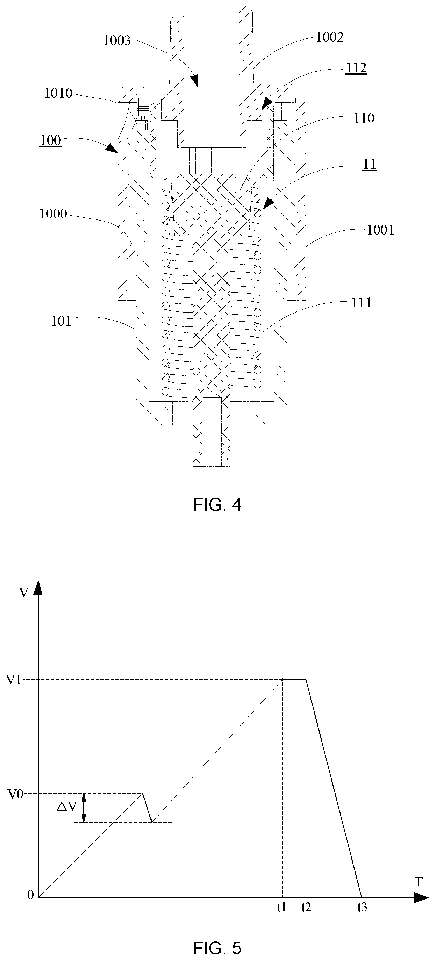

Refer to FIGs. 2 to 4, FIG. 2 shows a partial schematic structural diagram of an atomizer in an embodiment of the present application; FIG. 3 shows an exploded schematic structural diagram of the atomizer in the embodiment of the present application as shown in FIG. 2; and FIG. 4 shows a schematic cross-sectional structural diagram of the atomizer in the embodiment of the present application as shown in FIG. 3. As shown, the atomizer 1 includes an atomizer housing 10, a delivery mechanism 11, a cartridge (not shown), and a lower housing (not shown).

The cartridge is configured to accommodate multiple doses of the fluid, and the atomizer housing 10 is configured to receive the cartridge which can be inserted into the atomizer housing 10, e.g., in an arrow direction as shown in FIG. 2. The delivery mechanism 11 is configured to move from an initial position to a tensioning position when the atomizer is tensioned (also referred to as triggered or rotated or stretched) to withdraw a medication dosage of the fluid from the cartridge; and the delivery mechanism 11 is further configured to move from the tensioning position to the initial position when the atomizer is activated to convey and atomize the medication dosage of the fluid. The delivery mechanism 11 undergoes a temporary rebound motion during its movement from the initial position to the tensioning position, i.e., during the withdrawal of the medication dosage of the fluid, to convey and atomize the pre-determined amount of the fluid. The lower housing can be attached to/detached from the atomizer housing 10 to close/open the atomizer 1. Upon closing of the atomizer 1, the atomizer can be triggered or activated, and upon opening of the atomizer, the cartridge can be replaced or inserted. The temporary rebound motion means that the rebound motion occurs temporarily and will be blocked in a short time and the previous compression or tension motion will continue.

In an embodiment, the pre-determined amount is set to be positively correlated with the medication dosage, e.g., the pre-determined amount is set to be large when the medication dosage is large, and the pre-determined amount is set to be small when the medication dosage is small. In particular, the pre-determined amount can be set as 1 % to 15% of the medication dosage; preferably, it can be set as 3% to 8% of the medication dosage; e.g., the pre-determined amount can be set as about 1%, 2%, 3%, 4%, 5%, 6%, 7%, 8%, 9%, 10%, 11%, 12%, 13%, 14%, or 15%, etc. of the medication dosage.

In an embodiment, the pre-determined amount is set to be correlated with the surface area of the part to be rinsed, e.g., the pre-determined amount can be set to be 5-10 times more than the amount required by the area of the part to be rinsed to ensure effective rinsing. For example, the pre-determined amount is configured to rinse a nozzle in the delivery mechanism of the atomizer and can be set to be 5 to 10 times more than the amount required by the inner area of the nozzle.

In an embodiment, the pre-determined amount is set as 0.15 to 3 µL. Preferably, it can be set as 1 to 2 µL, e.g., the pre-determined amount can be set as about 0.5 µL, 1 µL, 1.5 µL, 2 µL, 2.5 µL, or 3 µL, etc.

In an embodiment, the pre-determined volume may be configured to rinse (also referred to as prewash) the delivery mechanism or the conveying assembly of the delivery mechanism or the nozzle of the delivery mechanism so that the deposit, crystal, fouling, contaminant or the like can be discharged or separated prior to the atomization of the medication dosage. The pre-determined amount can also be used for other purposes, which is not limited here in the present application.

FIG. 5 shows a schematic diagram of the generation stage of the pre-determined amount of the fluid in an embodiment of the present application. As shown, the abscissa in the figure indicates a timeline of triggering and activating the atomizer, and the ordinate indicates an axial moving stroke of the delivery mechanism or the cartridge reflecting the withdrawn and released amounts of the fluid, wherein V1 corresponds to the medication dosage, and ΔV corresponds to the pre-determined amount. As shown in FIG. 5, Stage 0 to t1 involves the triggering stage of the atomizer, in which the delivery mechanism 11 moves from the initial position to the tensioning position to generate a compression amount which can exactly allow the delivery mechanism to withdraw and store the medication dosage of the fluid. This stage involves three processes, wherein the first process is to tension the atomizer so that the atomizer withdraws a fluid less than the medication dosage (e.g., V0 in FIG. 5), the second process is a temporary rebound motion to atomize the pre-determined amount △V of the fluid, and the third process is to go ahead with tensioning the atomizer to the tensioning position to withdraw the medication dosage V1 of the fluid.

In other words, as shown in FIG. 5, the process of conveying and atomizing the pre-determined amount of the fluid occurs during the withdrawal of the medication dosage. The withdrawal of the medication amount of the fluid can be directly achieved, as long as it can be ensured that the initial position and the tensioning position correspond to the medication dosage. It greatly improves the tolerance to the accuracy of the pre-determined amount, while ensuring the accuracy of the medication amount. As an example, the pre-determined amount of the fluid is mainly for rinsing the nozzle, and the accuracy requirement of the pre-determined amount is much lower than that of the medication dosage. Even if there is a deviation in the pre-determined amount ΔV as shown in FIG. 5 in the design or during a long-term use, it will not affect the fact that the medication amount of the fluid can be finally withdrawn. In addition, as shown in FIG. 5, the release of the pre-determined amount of the fluid occurs automatically during the process of withdrawing the fluid by the atomizer, i.e., during the process that the patient/user still rotates or twists the atomizer, which would not cause any misunderstanding of the patient/user.

Hereinafter the operational principle and configuration of the atomizer will be illustrated by references to FIGs. 2 to 11.

In an embodiment, the cartridge is configured to store the atomized fluid and provide the fluid containing a pre-determined dose unit. For example, the cartridge includes 60 dose units of fluid, that is, the fluid in an amount that allows the atomizer 1 to spray 60 medication dosages of the fluid. The cartridge is substantially a cylindrical or box-shaped rigid structure, and includes a collapsible bag for storage of the fluid therein.

In an embodiment, the fluid is designed as a liquid containing an active substance that forms, when atomized, an aerosol (also referred to as soft mist, aerosol, fine particles, fine droplets, etc.) which can be breathed or inhaled by the patient/user. In some other embodiments, the fluid can include particles or powders, and the fluid can also be a liquid for cosmetics, suspension, or the like.

In an embodiment, the active substance of the fluid can include at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors, etc.

Still refer to FIGs. 2 to 4, the atomizer housing 10 includes an upper housing part 100 and an inner housing part 101 which is rotatable relative to the upper housing part 100. The lower housing (not shown) can be further attached to/detached from the inner housing part 101, and is rotatable relative to the upper housing part 100. Through the rotational motion of the lower housing relative to the atomizer housing 10 (e.g., the rotational motion produced by manual operation of the user/patient), the inner housing part 101 is carried to rotate relative to the upper housing part 100, so that the atomizer is triggered or enters a ready-to-use stage (also referred to as being ready for next use). The delivery mechanism 11 is coupled with the atomizer housing 10 and the cartridge so that the rotational motion of the lower housing relative to the upper housing part 100 (or also referred to as the rotational motion of the inner housing part 101 relative to the upper housing part 100) is converted to the downward axial movement of the cartridge in the atomizer 1. During the downward axial movement of the cartridge, the delivery mechanism 11 withdraws the fluid stored in the cartridge.

The axial movement refers to a movement in the axial direction of the atomizer, and the axis of the atomizer is as shown by the line X in FIG. 2. The axial movement may be in two directions, wherein the direction towards the atomizer housing 10 can be defined as an upward axial movement, and the direction away from the atomizer housing 10 can be defined as a downward axial movement. The axial movement as mentioned above and below can be similarly understood, which are not reiterated here.

One rotational motion of the inner housing part 101 relative to the upper housing part 100 can trigger the atomizer. The rotational angle of one rotational motion corresponds to the rotational angle required by triggering the atomizer, which can also be understood as the rotational angle required by the atomizer from the untriggered state to the ready-to-use state. For example, the one rotational motion may be a 180° rotational motion centered on the axis of the atomizer. During this process, the delivery mechanism 11 moves from the initial position to the tensioning position, so that the cartridge moves in the atomizer 1 axially downwards to the bottom to withdraw the medication dosage of the fluid from the cartridge. The initial position refers to a position of the delivery mechanism in a natural state in the atomizer when the atomizer has not been triggered. FIG. 4 shows the initial position of the delivery mechanism 11. The tensioning position refers to a position of the delivery mechanism in the atomizer where the delivery mechanism is sufficiently tensioned to withdraw the medication dosage of the fluid. The tension amount generated by moving from the initial position to the tensioning position can correspond to the withdrawal of the medication dosage of the fluid.

It should be understood that the initial position and the tensioning position are used to reflect the relative forms of the delivery mechanism in the atomizer before and after being triggered, respectively, but do not represent an absolute position. Since the delivery mechanism includes multiple components or parts or structures, the components of the delivery mechanism are not required or necessary to present the same forms in the same position when it is in the initial position or in the tensioning position. Hereinafter, when a particular component of the delivery mechanism 11 is described, the initial position and the tensioning position of the component should be similarly construed, which is not reiterated below.

In an embodiment as shown in FIG. 3 and FIG. 4, the inner wall of the upper housing part 100 is provided with an annular protruding portion 1000, while the outer wall of the inner housing part 101 is configured as a structure which cooperates with the annular protruding portion 1000 so that the inner housing part 101 can be attached to the upper housing part 100 and rotate relative to the upper housing part 100. FIG. 3 and FIG. 4 are only provided by way of example and should not be construed to limit the structures of the upper housing part 100 and the inner housing part 101, as long as their structures are designed by those skilled in the art to allow a relative rotation between them.

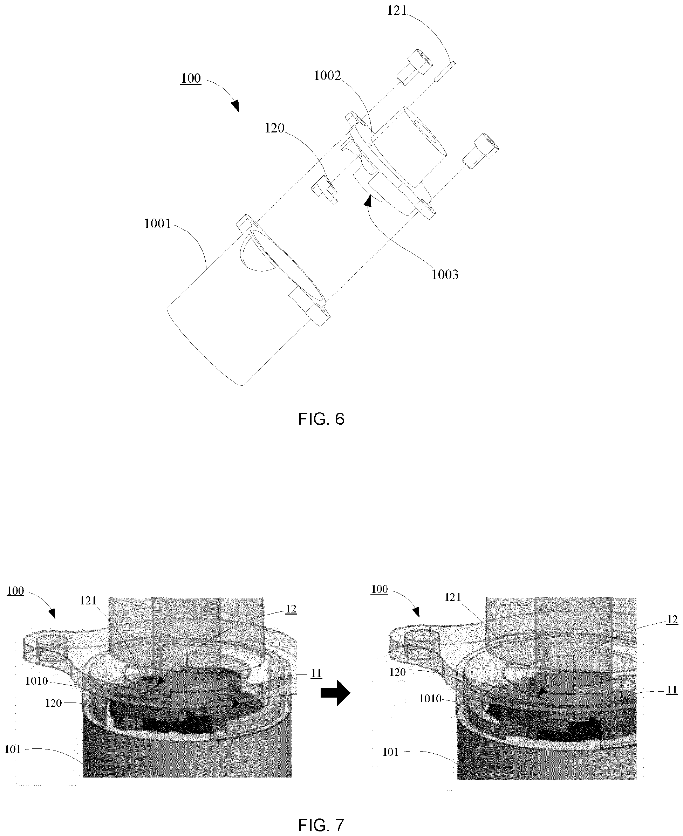

In an embodiment, FIG. 6 shows an exploded schematic structural diagram of the upper housing part in an embodiment of the present application. As shown, the upper housing part 100 includes a base 1001 and a suction nozzle 1002. The base 1001 is configured to support the inner housing part 101. For example, an annular protruding portion 1000 is further provided on the base 1001, and the inner housing part 101 is supported by the annular protruding portion 1000. The suction nozzle 1002 is attached to the base 1001, e.g., arranged on the base 1001 by means of screw fixation, and the patient/user can breathe/inhale the aerosol by the suction nozzle 1002.

In an embodiment as shown in FIG. 4 and FIG. 6, the suction nozzle 1002 is formed with a supply channel 1003 which can be configured to accommodate/configure/form some components or structures of the delivery mechanism, e.g., a microfluidic structure, a pressure chamber, a quantitative cavity of the delivery mechanism or the like. The structure of the delivery mechanism will be described in detail below.

To facilitate the protection of the suction nozzle 1002, in some embodiments, the suction nozzle 1002 can also be provided with an openable or closable cap (not shown), which can be fully or partially transparent so that the patient/user can monitor the discharge of the pre-determined amount of the fluid. It can also be designed to be opaque so that the patient/user cannot notice the discharge of the pre-determined amount of the fluid.

In an embodiment as shown in FIG. 2 and FIG. 6, the atomizer housing 100 is also provided with a rotatable blocking member 12. For example, the blocking member 12 is rotatably arranged on the suction nozzle 1002 of the atomizer housing 100. In some examples, the blocking member 12 includes a blocking portion 120 and a shaft 121. The shaft 121 is fixed onto the suction nozzle 1002, and the blocking portion 120 is attached to the shaft 121 and can rotate around the shaft 121.

During the above rotational motion of the inner housing part 101 relative to the upper housing part 100, the blocking member 12 is pushed to rotate to allow part of the blocking part 120 of the blocking member 12 to enter an inner space of the inner housing part 101 and be further located in the moving path of the delivery mechanism 11. As a result, the blocking member 12 can block the delivery mechanism 11 after one rotational motion and contacting with each other or after the triggering of the atomizer, so that the atomizer is in a ready-to-use state, thus avoiding the atomizer from automatically conveying and atomizing the medication dosage of the fluid without the activation by the patient/user.

Referring to FIG. 7 in combination with FIGs. 3 to 7, FIG. 7 shows a schematic process diagram of a blocking member being pushed in an embodiment of the present application. As shown in FIGs. 3 to 7, a protruding portion 1010 is arranged on the inner housing part 101. During the rotational motion of the inner housing part 101 relative to the upper housing part 100, the protruding portion 1010 also rotates relative to the upper housing part 100, and during the rotation, the protruding portion 1010 will contact the blocking member 12 on the upper housing part 100 and push the blocking member 12 to rotate. In particular, the protruding portion 1010 of the inner housing part 101 has a slope surface in consistence with a direction of its rotational motion (for conforming to the shape of the inner housing part 101, the slope surface may have a certain radian). During the rotation, the slope surface of the protruding portion 1010 contacts the blocking portion 120 of the blocking member 12 to form a movement track of the blocking portion 120, driving the blocking member 120 to rotate around the shaft 121.

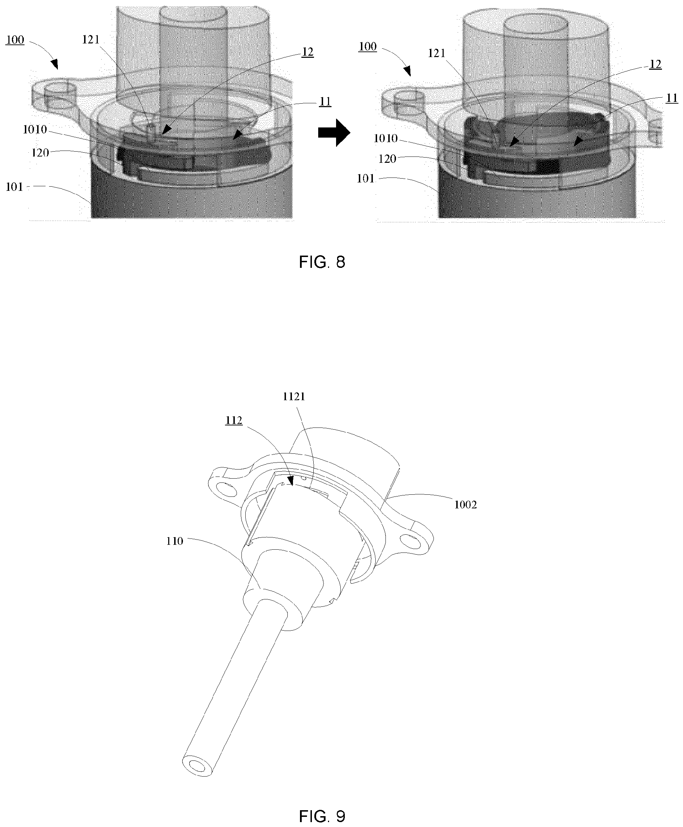

The blocking member 12 is also configured to be rotated by manual operation to remove the block on the delivery mechanism 11, thereby activating the atomizer. After removing the block of the blocking member 12 on the delivery mechanism 11, the delivery mechanism 11 will move from the tensioning position to the initial position (while allowing the cartridge to move axially upwards from the bottom to the initial position in the atomizer) to convey and atomize the medication dosage of fluid. Referring to FIG. 8 in combination with FIGs. 3 to 5, FIG. 8 shows a schematic process diagram of removing the block of the blocking member on the delivery mechanism in an embodiment of the present application. As shown, when the patient/user needs to activate the atomizer, he/she can manually actuate (such as, press) the blocking member 12 to allow the blocking member 12 rotate in a direction opposite to the pushing direction as shown in FIG. 6, so that the blocking member 12 is away from the moving path of the delivery mechanism 11 and cannot block the delivery mechanism 11, that is, the delivery mechanism 11 is released so that the medication dosage of the fluid withdrawn in the triggering stage of the atomizer can be conveyed and atomized.

In an embodiment as shown in FIG. 3 and FIG. 4, the delivery mechanism 11 includes a cartridge translator 110, a drive spring 111, and a transmission structure 112. The cartridge translator 110 is configured to connect the cartridge to fix a fully inserted cartridge in the atomizer and drive the cartridge to move in the axial direction. The drive spring 111 is coupled with the cartridge translator 110. The transmission structure 112 is configured to cooperate with the cartridge translator 110 to convert the rotational motion when the atomizer is triggered to an axial movement. For example, the drive spring 111 can be supported in the atomizer housing 10 by the inner housing part 101, the cartridge translator 110 is inserted into and abuts against the drive spring 111, and the transmission structure 112 cooperates with the cartridge translator 110 upon the rotation of the inner housing part 101 relative to the upper housing part 100 (also called upon the triggering of the atomizer), so that the drive spring 111 is compressed downwards (also called tensioned) by the cartridge translator 110 to the tensioning position where the cartridge translator 110 is blocked by the blocking member 12, while the drive spring 111 is maintained at the tensioning position. After the patient/user manually operates the blocking member 12 to remove the block on the cartridge translator 110, the drive spring 111 releases an elastic force to drive the cartridge translator 110 to move upwards to the initial position.

In an embodiment, the delivery mechanism 11 can further include a delivery assembly (not shown) including, e.g., a conveying tube, a non-return valve, a pressure chamber, a nozzle, etc. The nozzle can include a microfluidic structure which can disperse the fluid into aerosol. The components in the delivery assembly can cooperate with each other to perform the withdrawal, delivery, atomization of the fluid. The conveying tube is arranged on the cartridge translator 110 and passed into the cartridge for communication with the fluid in the cartridge when the cartridge is inserted into the atomizer housing 10. When the atomizer is triggered to tension the drive spring 111, the cartridge translator 110, along with the cartridge and the conveying tube, moves downwards, and the fluid in the cartridge is sucked out of the cartridge via the non-return valve and enters the pressure chamber. After the manual operation of the blocking member 12, the drive spring 111 releases an elastic force so that the conveying tube, along with its non-return valve in closed mode, moves towards the pressure chamber where the fluid is under pressure so that the fluid in the pressure chamber is forced to atomize and output via the nozzle.

The transmission structure 112 as mentioned above can convert the rotational motion when triggering the atomizer into an axial movement, wherein, during the one rotational motion triggering the atomizer (i.e., one rotational motion of the inner housing part 101 relative to the upper housing part 100), the axial movement includes a first moving stroke and a second moving stroke, and the aforementioned temporary rebound motion generated by the delivery mechanism during its movement from the initial position to the tensioning position occurs during switching from the first moving stroke to the second moving stroke.

Refer to FIG. 5 and the description thereof, when rotating the atomizer by a patient/user, in Stage 0 to t1, one rotational motion of the inner housing part 101 relative to the upper housing part 100 occurs, which will be converted to an axial movement of the cartridge translator 110 or the cartridge, namely, corresponding to a tensioning motion of the drive spring 111. The first moving stroke contained in the axial movement corresponds to Stage 0 to V0 in the ordinate in FIG. 5 and enables the atomizer to withdraw a fluid in an amount less than the medication dosage; the second moving stroke contained in the axial movement corresponds to Stage V0-△V to V1 in the ordinate in FIG. 5 and enables the atomizer to go ahead with withdrawing the fluid until the medication dosage V1; and the temporary rebound motion corresponds to Stage V0 to V0-△V and occurs during switching from the first moving stroke to the second moving stroke.

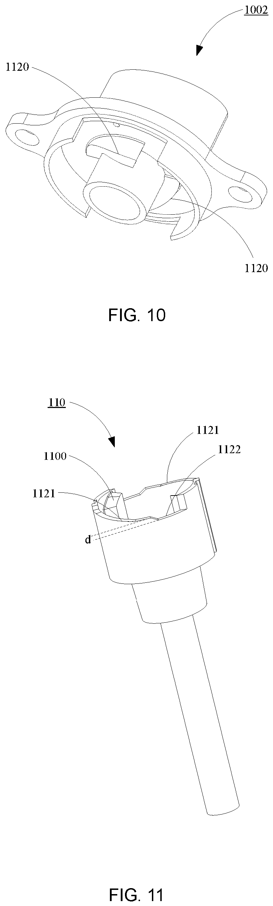

Referring to FIGs. 9 to 11 in combination with FIGs. 3 to 4, FIG. 9 shows a schematic structural diagram of the suction nozzle in combination with the cartridge translator in an embodiment of the present application, FIG. 10 shows a three-dimensional schematic structural diagram of the suction nozzle in an embodiment of the present application, and FIG. 11 shows a three-dimensional schematic structural diagram of the cartridge translator in an embodiment of the present application, wherein the transmission structure 112 includes a first track 1120 and a second track 1121.

In an embodiment, the first track 1120 includes an inclined surface (also called a slope surface) formed on the atomizer housing 10, and further, the inclined surface is formed on the suction nozzle 1002 of the upper housing part 100. The inclined surface of the first track 1120 contacts the cartridge translator 110 to achieve the first moving stroke of the axial movement. The cartridge translator 110 is provided with a guide structure 1100 which can move on the first track 1120 to achieve the first moving stroke of the axial movement. In particular, during the rotational motion for triggering the atomizer, that is, the inner housing part 101 rotates relative to the upper housing part 100, driving the cartridge translator 110 to rotate relative to the upper housing part 100, so that the guide structure 1100 on the cartridge translator 110 travels on the inclined surface of the first track 1120. Due to the slope of the inclined surface, the cartridge translator 110 is driven to move downwards axially during traveling, and thus the movement of the guide structure 1100 on the first track 1120 can be converted into the first moving stroke.

In an embodiment, the second track 1121 is formed on the cartridge translator 110, and after the cartridge translator 110 is detached from the first track 1120, the second track 1121 contacts with the blocking member 12 to achieve the second moving stroke of the axial movement. As shown in FIG. 11, the initial position of the second track 1121 is provided with a groove 1122, which, when the cartridge transalator 110 is detached from the first track 1120, allows the drive spring 111 to release some elastic force to drive the cartridge translator 110 to move upwards axially until the cartridge translator 110 is blocked by the blocking member, thereby forming the rebound motion. In particular, during the rotation of the inner housing part 101 relative to the upper housing part 100 to facilitate the movement of the guide structure 1100 of the cartridge translator 110 on the first track 1120, the blocking member 12 is rotated in a manner as shown in FIG. 7 and the description thereof. After the inner housing part 101 rotates relative to the upper housing part 100 until the guide structure 1100 of the cartridge translator 110 moves to the end of the first track 120, it will be detached from the first track 1120. Because the distance between the blocking member 12 and the second track 1121 is the depth d of the groove 1122, the drive spring 111 can automatically release some elastic force to drive the cartridge translator 110 to move upwards axially the distance d to contact with the blocking member 12. During this process, the atomizer outputs and atomizes a pre-determined amount of the fluid. At that time, the inner housing part 101 has not yet rotated relative to the upper housing part 100 to the tensioning position, and the rotational motion will cause the blocking member 12 to move relatively on the second track 1121. Since the blocking member 12 moves relatively on a slope surface of the second track 1121, it facilitates the cartridge translator 110 further move downwards axially. Hence, by the contact and relative motion of the blocking member 12 with the second track 1121, the second moving stroke can be achieved.

In an embodiment, the transmission structure 112 includes at least two first tracks 1120 and at least two second tracks 1121. The at least two first tracks 1120 are rotationally symmetrical, and the at least two second tracks 1121 are rotationally symmetrical, too. It should be understood that the specific number of the first tracks 1120 and the second tracks 1121 can be selected according to the rotational angle of one rotational motion of the atomizer. One first track 1120 and one second track 1121 can achieve the conversion of one rotational motion into an axial movement. For example, as shown in FIGs. 3 to 11, the one rotational motion of the atomizer has a rotational angle of 180°. In the embodiment, it is configured to have two first tracks 1120 and two second tracks 1121, wherein one of the first tracks 1120 cooperates with one of the second tracks 1121 to convert the one rotational motion into an axial movement, so that the atomizer enters the ready-to-use state. After the atomizer conveys and atomizes the medication dosage of the fluid, if the atomizer is further rotated next time, the other of the first tracks 1120 will cooperate with the other of the second tracks 1121 to achieve the conversion of that rotational motion to an axial movement.

Those skilled in the art can also configure one first track 1120 and one second track 1121 according to the inspiration of the present application in combination with practical requirements, so that the one rotational motion needs to be correspondingly configured to have a rotational angle of 360°, and the patient/user has to make a 360 ° rotation to complete the operation of triggering the atomizer.

In some embodiments, the atomization of the pre-determined amount of the fluid as described above can take place at any time point during the movement of the delivery mechanism from the initial position to the tensioning position, that is, it can take place at any time point during the withdrawal of the medication dosage of the fluid. In view of this, the stroke length of the guide structure 1100 of the cartridge translator 110 provided by the first tracks 1120, as well as the stroke length of the blocking member 12 provided by the second tracks 1121 as shown in FIGs. 8 to 9 and the description thereof can be set in accordance with the timing for atomization of the pre-determined amount of the fluid. By changing the stroke length of the guide structure 1100 of the cartridge translator 110 provided by the first tracks 1120 and the stroke length of the blocking member 12 provided by the second tracks 1121, those skilled in the art can correspondingly change the timing for atomization of the pre-determined amount of the fluid.

In an embodiment, the depth d of the groove 1122 arranged on the second track 1121 is positively correlated with the pre-determined amount. Namely, in the example that the pre-determined amount is larger, the depth d needs to be correspondingly set larger, and in the example that the pre-determined amount is smaller, the depth d needs to be set smaller. In view of the fact that the volume specifically set for the pre-determined amount is positively correlated with the volume of the medication dosage, the depth d of the groove 1122 can also be described as being positively correlated with the medication dosage. Those skilled in the art can adjust the depth d of the groove 1122 as required.

In an embodiment, the depth d of the groove 1122 is set as 0.1 to 1.5 mm. Preferably, it can be set as 0.4 to 0.8 mm. For example, the depth d of the groove 1122 can be set as about 0.1 mm, 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, or 1.5 mm, etc.

In some embodiments, the atomizer can also include other components, such as, a counting device for counting the activation of the atomizer by detecting the rotation of the inner housing part 101 relative to the upper housing part 100, and for detecting the number of the inserted cartridges. Further, the counting device can also be coupled with a locking element to prevent the further use of the atomizer, e.g., to prevent the inner housing part 101 from rotating relative to the upper housing part 100 when a certain number of activation or operation or release of the medication dosage has been reached or exceeded.

In a word, the atomizer for a fluid disclosed in the present application conveys and atomizes a pre-determined amount of the fluid during the withdrawal of a medication dosage of the fluid by the atomizer, thereby avoiding any potential health hazard or wrong guidance brought by the atomizer to a patient/user, while ensuring the accuracy of the medication dosage.

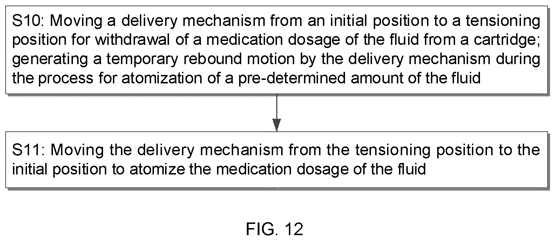

In another aspect, the present application further discloses a method for conveying and atomizing a fluid for use in the atomizer for the fluid as described above. The atomizer as described in any one of the embodiments in FIGs. 1 to 11 and the description thereof can be used as the atomizer. FIG. 12 shows a flowchart of a method for conveying and atomizing a fluid in an embodiment of the present application. As shown, the method for conveying and atomizing the fluid includes Step S10 and Step S11.

In Step S10, the delivery mechanism is moved from the initial position to the tensioning position to withdraw a medication dosage of the fluid from the cartridge.

Refer to the description of FIGs. 2 to 8, the atomizer utilizes a structure as described in any one of the embodiments in FIGs. 2 to 8 to execute Step S10. Referring to the description of FIGs. 2 to 8 for the specific structure and the detailed process of Step S10, which are not iterated here.

In Step S10, the delivery mechanism generates a temporary rebound motion during its movement from the initial position to the tensioning position to atomize a pre-determined amount of the fluid. Referring to FIGs. 9 to 11 in combination with the description of FIGs. 2 to 8, the atomizer utilizes a structure described in any one of the embodiments in FIGs. 2 to 11 to generate the rebound motion and atomize the pre-determined amount of the fluid. Refer to the description of FIGs. 2-11 for the detailed process, which is not iterated here.

The medication dosage refers to a single medication dosage, which is an amount capable of producing a therapeutic effect on the patient/user. The pre-determined amount refers to a pre-designed amount according to its use, which can be pre-designed by those skilled in the art according to its practical use. Typically, the pre-determined amount is less than the medication dosage.

In an embodiment, the pre-determined amount is set to be positively correlated with the medication dosage, e.g., the pre-determined amount is set to be large when the medication dosage is large, and the pre-determined amount is set to be small when the medication dosage is small. In particular, the pre-determined amount can be set as 1 % to 15% of the medication dosage; preferably, it can be set as 3% to 8% of the medication dosage; e.g., the pre-determined amount can be set as about 1%, 2%, 3%, 4%, 5%, 6%, 7%, 8%, 9%, 10%, 11%, 12%, 13%, 14%, or 15%, etc. of the medication dosage.

In an embodiment, the pre-determined amount is set to be correlated with the area of the part to be rinsed, e.g., the pre-determined amount can be set to be 5-10 times more than the amount required by the area of the part to be rinsed to ensure effective rinsing. For example, the pre-determined amount is configured to rinse a nozzle in the delivery mechanism of the atomizer and can be set to be 5 to 10 times more than the amount required by the inner area of the nozzle.

In an embodiment, the pre-determined amount is set as 0.15 to 3 µL, preferably, it can be set as 1 to 2 µL, e.g., the pre-determined amount can be set as about 0.5 µL, 1 µL, 1.5 µL, 2 µL, 2.5 µL, or 3 µL, etc.

In an embodiment, the pre-determined amount may be used to rinse (also referred to as prewash) the delivery mechanism or the conveying assembly of the delivery mechanism or the nozzle of the delivery mechanism so that the deposit, crystal, fouling, contaminant or the like can be discharged or separated prior to the atomization of the medication dosage. The pre-determined amount can also be used for other purposes, which is not limited here in the present application.

In an embodiment, the fluid is designed as a liquid including an active substance that forms, when atomized, an aerosol (also referred to as soft mist, aerosol, fine particles, fine droplets, etc.) which can be breathed or inhaled by the patient/user. In some other embodiments, the fluid can include particles or powders, and the fluid can also be a liquid for cosmetics, suspension, or the like.

In an embodiment, the active substance of the fluid can include at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors.

In Step S11, the delivery mechanism is moved from the tensioning position to the initial position to atomize the medication dosage of the fluid.

Refer to the description of FIGs. 2 to 11, the atomizer utilizes a structure described in any one of the embodiments in FIGs. 2 to 11 to execute Step S11. Referring to the description of FIGs. 2 to 11 for the specific structure and the detailed process of Step S10, which are not iterated here.

In summary, the method for conveying and atomizing a fluid disclosed in the present application conveys and atomizes a pre-determined amount of the fluid during the withdrawal of a medication dosage of the fluid by the atomizer, thereby avoiding any potential health hazard or wrong guidance brought by the atomizer to a patient/user, while ensuring the accuracy of the medication dosage.

The above examples are merely illustrative of the principle and efficacy of the application and are not intended to limit the application. Any skilled person can modify or change the aforementioned examples without departing from the spirit and scope of the present application. Therefore, all equivalent modifications or changes made by those of ordinary skills in the art to which the application belongs without departing from the spirit and technical ideas disclosed in the application shall still be covered by the appended claims of the application.

## Claims

1. An atomizer for a fluid, comprising:
a cartridge for accommodating multiple doses of the fluid; and
a delivery mechanism movable from an initial position to a tensioning position for withdrawal of a medication dosage of the fluid from the cartridge when the atomizer is tensioned, and movable from the tensioning position to the initial position for atomization of the medication dosage of the fluid when the atomizer is activated;
wherein a pre-determined amount of the fluid is atomized by a temporary rebound motion of the delivery mechanism during its movement from the initial position to the tensioning position, and wherein the medication dosage is a single medication dose, and the pre-determined amount is less than the medication dosage.

2. The atomizer according to claim 1, further comprising:
an atomizer housing for receiving the cartridge; and
a lower housing for opening the atomizer to replace or insert the cartridge, wherein a rotational motion of the lower housing relative to the atomizer housing enables the atomizer to be tensioned or ready for use.

3. The atomizer according to claim 1, wherein the delivery mechanism comprises:
a cartridge translator for connecting with the cartridge and driving the cartridge to move in an axial direction;
a drive spring coupled with the cartridge translator for driving the cartridge translator back to the initial position when an elastic force is released; and
a transmission structure for cooperating with the cartridge translator to convert a rotational motion of the atomizer when being tensioned into an axial movement, wherein during one rotational motion, the axial movement comprises a first moving stroke and a second moving stroke, and the temporary rebound motion occurs during switching from the first moving stroke to the second moving stroke.

4. The atomizer according to claim 3, wherein the one rotational motion is a 180° rotational motion centered on an axis of the atomizer.

5. The atomizer according to claim 3, wherein the transmission structure comprises:
a first track comprising an inclined surface formed on the atomizer housing of the atomizer, the inclined surface being in contact with the cartridge translator to achieve the first moving stroke of the axial movement; and
a second track formed on the cartridge translator, the second track being in contact with a blocking member after the cartridge translator is detached from the first track to achieve the second moving stroke of the axial movement.

6. The atomizer according to claim 5, wherein the blocking member is rotatably arranged on the atomizer housing and pushed to rotate to contact the second track during the rotational motion.

7. The atomizer according to claim 6, wherein the atomizer housing of the atomizer comprises an upper housing part and an inner housing part, wherein the inner housing part is rotatably supported on the upper housing part, and a protruding portion is provided on the inner housing part to push the blocking member to rotate during the rotational motion.

8. The atomizer according to claim 5, wherein the blocking member is further configured to be rotated by manual operation to release the cartridge translator to allow the drive spring to release an elastic force.

9. The atomizer according to claim 5, wherein the transmission structure comprises at least two first tracks and at least two second tracks.

10. The atomizer according to claim 5, wherein a groove is provided at an initial position of the second track, wherein when the cartridge translator is detached from the first track, the groove allows the drive spring to release some elastic force to drive the cartridge translator to move upwards until the cartridge translator is blocked by the blocking member, thereby forming the rebound motion.

11. The atomizer according to claim 10, wherein a depth of the groove is positively correlated with the pre-determined amount or the medication dosage.

12. The atomizer according to claim 10, wherein a depth of the groove ranges from 0.1 to 1.5 mm.

13. The atomizer according to claim 5, wherein the cartridge translator comprises a guide structure which moves on the first track to achieve the first moving stroke of the axial movement.

14. The atomizer according to claim 1, wherein the pre-determined amount is used to rinse a nozzle of the atomizer.

15. The atomizer according to claim 1, wherein the pre-determined amount ranges from 0.15 to 3 µL.

16. The atomizer according to claim 1, wherein the pre-determined amount is between 1% and 15% of the medication dosage.

17. The atomizer according to claim 1, wherein the fluid comprises an active substance.

18. The atomizer according to claim 17, wherein the active substance comprises at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors.

19. A method for conveying and atomizing a fluid, comprising:
moving a delivery mechanism from an initial position to a tensioning position for withdrawal of a medication dosage of the fluid from a cartridge;
generating a temporary rebound motion by the delivery mechanism during the movement from the initial position to the tensioning position for atomization of a pre-determined amount of the fluid; and
moving the delivery mechanism from the tensioning position to the initial position to atomize the medication dosage of the fluid;
wherein the medication dosage is a single medication dose, and the pre-determined amount is less than the medication dosage.

20. The method for conveying and atomizing the fluid according to claim 19, wherein the pre-determined amount is configured to rinse a nozzle of the atom izer.

21. The method for conveying and atomizing the fluid according to claim 19, wherein the pre-determined amount ranges from 0.15 to 3 µL.

22. The method for conveying and atomizing the fluid according to claim 19, wherein the pre-determined amount is between 1% and 15% of the medication dosage.

23. The method for conveying and atomizing the fluid according to claim 19, wherein the fluid comprises an active substance.

24. The method for conveying and atomizing the fluid of claim 23, wherein the active substance comprises at least one of anticholinergics, beta receptor agonists, steroids, phosphodiesterase-IV-inhibitors, LTD4-antagonists, EGFR-kinase inhibitors, antiallergic drugs, ergot alkaloid derivatives, triptans, CGRP-antagonists, and phosphodiesterase-V-inhibitors.
